# EUROPÄISCHE PATENTSCHRIFT

(11) **EP 1 816 462 B1**
(45) Veröffentlichungstag und Bekanntmachung des Hinweises auf die Patenterteilung: **06.04.2011**
(21) Anmeldenummer: 06101329.8
(22) Anmeldetag: 06.02.2006
(51) Int. Cl.: G01N 21/15, G01N 33/18

(54) **Abwasser-Tauchsonde**
Wastewater immersion probe
Sonde immergée pour eaux usées

(43) Veröffentlichungstag der Anmeldung: 08.08.2007
(73) Patentinhaber: Hach Lange GmbH, 14163 Berlin (DE)
(72) Erfinder: Battefeld, Manfred, 40211, Düsseldorf (DE); Jonak, Andreas, 40667, Meerbusch (DE); Heidemanns, Lothar, 41352, Korschenbroich (DE); Leyer, Axel, 41199, Mönchengladbach (DE); Schuster, Michael, 41462, Neuss (DE)
(74) Vertreter: Ter Smitten, Hans

(56) Entgegenhaltungen:
- EP-A- 0 590 487
- EP-A- 0 634 645
- ANONYMUS: "Firmenprospekt "Prozess-Messtechnik" der Firma Hach Lange GmbH, Teil 4."[Online] Januar 2004 (2004-01), Seiten 83-133, XP002382972 Gefunden im Internet: URL:http://www.hach-lange.de/common/docume nts/1005/1009/10544_Ebook_D_HL_Katalog_Tei l4.pdf> [gefunden am 2006-05-29]

## Beschreibung

Die Erfindung bezieht sich auf eine Abwasser-Tauchsonde mit einem flüssigkeitsdichten Gehäuse mit einem Sensorfenster und einem Wischer zur außenseitigen Reinigung des Sensorfensters.

Abwasser-Tauchsonden werden zur Ermittlung verschiedener Parameter im Abwasser eingesetzt. Beispiele hierfür sind Trübungs- oder Feststoffsonden, Ultraschall-Schlammspiegelsonden, UV- oder Nitratsonden etc. In diesen Tauchsonden ist jeweils in dem Gehäuse ein Sensor angeordnet, der durch ein Sensorfenster in bzw. an dem Gehäuse einen der genannten Parameter in dem Abwasser außerhalb des Gehäuses misst. Ablagerungen an der Außenseite des Sensorfensters können Messungen des Sensors verfälschen oder unmöglich machen.

Aus einem Firmenprospekt "Prozess-Messtechnik" der Hach Lange GmbH Berlin, Januar 2004, Seite 103, ist es bekannt, das Sensorfenster mit einem Wischer zu reinigen.

Aus DE 42 33 218 A1 und aus EP 0 590 487 A1 ist jeweils eine Abwasser-Tauchsonde zur Trübungsmessung bekannt. Der Sensor ist ein optischer Sensor, der durch ein optisches Fenster hindurch Licht empfängt. Das Sensorfenster kann durch einen Wischer gereinigt werden, der außenseitig über das Sensorfenster streicht. Der Wischer wird durch einen in dem Gehäuse angeordneten elektrischen Antriebsmotor angetrieben. Die Verbindung des Antriebsmotors mit dem Wischer erfolgt durch eine Welle, die durch eine Öffnung des Gehäuses hindurchtritt. Die Welle muss daher mit einer Wellendichtung abgedichtet werden. Wellendichtungen sind erfahrungsgemäß nur begrenzt dichtend bzw. nur begrenzte Zeit zuverlässig dichtend. Im Falle einer Undichtigkeit dringt Flüssigkeit in das Gehäuse ein, so dass das Gehäuse schließlich geflutet und die Komponenten darin zerstört werden.

Aufgabe der Erfindung ist es dem gegenüber, eine Abwasser-Tauchsonde mit verbesserter Abdichtung zu schaffen.

Diese Aufgabe wird erfindungsgemäß gelöst mit einer Abwasser-Tauchsonde mit den Merkmalen des Patentanspruches 1.

Die erfindungsgemäße Abwasser-Tauchsonde weist ein öffnungsfreies Gehäuse auf, d. h. sie weist jedenfalls keine Öffnung mehr für eine Antriebswelle auf. Sowohl dem Antriebsmotor als auch dem Wischer ist jeweils ein magnetisches Element zugeordnet, durch die der Antriebsmotor mit dem Wischer zur Übertragung von Drehmomenten gekoppelt ist. Das magnetische Element des Antriebsmotors erzeugt ein die nicht-metallische Gehäusewand durchdringendes Magnetfeld, das das magnetische Element des Wischers bewegen bzw. mitnehmen kann. Selbstverständlich ist auch eine kinematische Umkehr möglich, d. h. das magnetische Element des Wischers erzeugt ein Magnetfeld, wodurch der Wischer kraftmäßig mit dem betreffenden magnetischen Element des Antriebsmotors gekoppelt ist. Alternativ können auch sowohl die Elemente des Wischers als auch die des Antriebsmotors magnetisiert sein, d. h. ein Magnetfeld erzeugen.

Hierdurch wird eine berührungsfreie wellenlose mechanische Kopplung des Antriebsmotors mit dem Wischer realisiert, die eine Gehäuseöffnung für eine Antriebswelle entbehrlich macht. Durch den Wegfall der Antriebswellen-Gehäuseöffnung entfällt die Hauptgefahr für das Eindringen von Feuchtigkeit bzw. Flüssigkeit aus dem Abwasser in das Gehäuse. Hierdurch werden die Zuverlässigkeit und die durchschnittliche Lebensdauer der Abwasser-Tauchsonde erheblich vergrößert. Die magnetische Kopplung des Antriebsmotors mit dem Wischer stellt ferner eine Rutschkupplung dar, die das übertragbare Drehmoment begrenzt. Hierdurch wird der Wischer bei Kollisionen besser gegen Deformation und Beschädigung geschützt.

Eine passive magnetische Kopplung des Antriebsmotor-Elementes mit dem Wischer-Element kann grundsätzlich auf zwei verschiede Weisen realisiert werden. Einerseits kann die Kopplung über Dauermagnete an beiden Elementen erfolgen, die Kopplung kann jedoch auch über Dauermagnete an einem Element und ferromagnetische, nicht-magnetisierte Teile an dem anderen Element erfolgen.

Alternativ kann in einer aktiven magnetischen Variante das gehäuseseitige Element als Stator und das wischerseitige Element als permanent erregter Rotor eines Elektromotors ausgelegt sein, der den Antriebsmotor bildet.

Während die passiv-magnetische Kopplung konstruktiv relativ einfach ist und größtenteils mit Standard-Bauteilen realisiert werden kann, ist die aktivmagnetische Variante der Antriebsmotor-Wischer-Kopplung eine raumsparende Lösung, mit der problemlos auch große Drehmomente übertragen werden können, wenn erforderlich.

Gemäß einer bevorzugten Ausgestaltung ist die Wischerbewegung eine oszillierende Bewegung und weisen das Gehäuse außenseitig und der Wischer Anschlagelemente auf, die die oszillierende Wischbewegung mechanisch begrenzen. Durch die Anschlagelemente wird der Wischwinkel des Wischers zuverlässig begrenzt. Da die magnetische Kopplung des Antriebsmotors und des Wischers auf ein maximales Drehmoment begrenzt ist, kann die Kopplung bei Auftreten eines darüber liegenden Drehmomentes, das beispielsweise durch Verschmutzung, ein Verharken des Wischers oder andere äußere Einflüsse verursacht sein kann, zu einem "Schrittfehler" führen, so dass der Wischer anschließend nicht mehr oder nur noch teilweise das Sensorfenster wischt. Durch die Anschlagelemente wird sichergestellt, dass der Wischer nach einer kompletten Wischsequenz wieder in seine ursprüngliche Position zurückrastet, und wieder das gesamte Soll-Wischfeld bestreicht. Durch die Anschlagelemente wird ferner verhindert, dass der außer Tritt geratene Wischer überhaupt das festgelegte Wischfeld verlassen kann.

Gemäß einer bevorzugten Ausgestaltung weist der Wischer einen das magnetische Element bzw. die magnetischen Elemente enthaltenden Ringkörper auf, der auf einem gehäuseseitigen Topf sitzt bzw. diesen ringartig umgibt. Bei einer Ausbildung der magnetischen Antriebsmotor- und Wischer-Elemente als Elektromotor bildet der Topf einen sogenannten Spalttopf. Der Topf bzw. der Spalttopf besteht aus nicht-magnetischem Material.

Vorzugsweise ist dem Gehäuse ein Spoiler zugeordnet, der eine kreisförmige Spaltöffnung zwischen dem Wischer-Ringkörper und dem Gehäuse radial überdeckt. In die Spaltöffnung können grundsätzlich Feststoffe aus dem Abwasser geraten und dort eingeklemmt werden, insbesondere faserartige Feststoffe in vorbeifließendem Abwasser. Durch den Spoiler werden diese Feststoffe derartig abgelenkt, dass sie nicht in die umlaufende kreisförmige Spaltöffnung geraten. Hierdurch wird ein Eindringen von insbesondere faserartigen Feststoffen in die Spaltöffnung und ein hieraus resultierendes Verklemmen des Wischer-Ringkörpers an dem Gehäuse-Topf vermieden.

Vorzugsweise ist zwischen dem Wischer-Ringkörper und dem Gehäuse-Topf ein Radialspiel vorgesehen, wobei die radiale Führung des Wischer-Ringkörpers ausschließlich durch eine Verriegelungsscheibe erfolgt, die auf dem Topf axial zentral befestigt ist. Die Verriegelungsscheibe bildet mit dem Wischer-Ringkörper eine kreisringförmige Berührungslinie. Hierdurch ist die Radiallager-Fläche auf eine kleine Fläche begrenzt, so dass auch die Lagerreibung auf ein Minimum reduziert wird. Dies ist deshalb von besonderer Bedeutung, weil die magnetische Kopplung des Wischers mit dem Antriebsmotor nur ein relativ kleines Drehmoment übertragen kann.

Gemäß einer bevorzugten Ausgestaltung ist der Wischer-Ringkörper durch die magnetischen Elemente des Wischers und des Antriebsmotors axial vorgespannt. Vorzugsweise erfolgt die Vorspannung des Wischer-Ringkörpers in proximaler Richtung, also in Richtung des Gehäuses. In distaler Richtung verbleibt ein gewisses Spiel zwischen dem Wischer-Ringkörper und der Verriegelungsscheibe. Ein Verklemmen kann dadurch weitgehend ausgeschlossen werden. Durch die axiale Vorspannung zusammen mit einem gewissen Axialspiel des Wischer-Ringkörpers wird der Wischer mit einer definierten Kraft auf das Sensorfenster gedrückt. Falls der Wischer auf ein kleines Hindernis stößt, kann er dennoch unter Überwindung der axialen Vorspannkraft kippen und dem Hindernis auf diese Weise ausweichen. Die axiale Vorspannung ist so gewählt, dass nur relativ geringe axiale Reibungskräfte zwischen dem Wischer-Ringkörper und dem Gehäuse auftreten und eine stabile radiale Fixierung des Ringkörpers an der Verriegelungsscheibe erfolgt.

Gemäß einer bevorzugten Ausgestaltung weisen die Verriegelungsscheibe und der Topf Rastelemente auf, durch die die Verriegelungsscheibe in ihrer Verriegelungsposition an dem Topf verrastet ist. Durch die Verrastung ist die Verriegelungsscheibe gesichert gegen unbeabsichtigtes Lösen, beispielsweise durch Vibrationen u. ä.

Im Folgenden wird unter Bezugnahme auf die Zeichnungen ein Ausführungsbeispiel der Erfindung näher erläutert.
Es zeigen:
- Fig. 1: Eine perspektivische Unteransicht einer Abwasser-Tauchsonde mit einem magnetisch gekoppelten Wischer, und
- Fig. 2: einen Querschnitt der Abwasser-Tauchsonde der Figur 1.

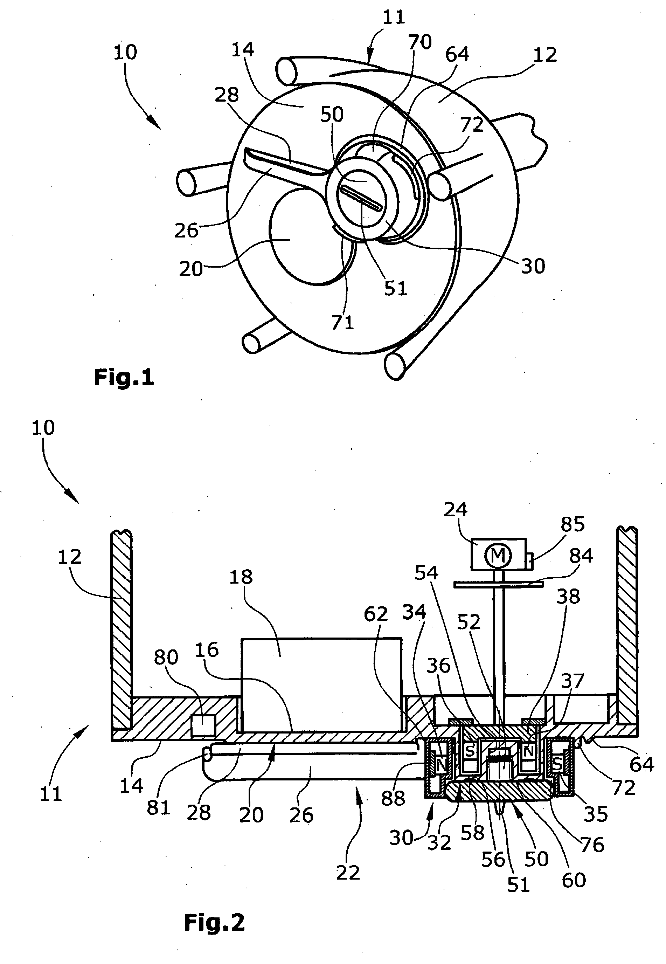

In den Figuren 1 und 2 ist eine Abwasser-Tauchsonde 10 dargestellt, die vorliegend als Ultraschall-Schlammspiegelsonde ausgebildet ist. Die Tauchsonde 10 weist ein zweiteiliges Gehäuse 11 auf, das von einem topfartigen Gehäusekörper 12 und einem kreisscheibenförmigen Gehäusedeckel 14 gebildet wird, der die untere Gehäusewand des Gehäuses 11 bildet. Der Gehäusekörper 12 besteht aus Metall, während der Gehäusedeckel 14 aus Kunststoff gefertigt ist. Der Gehäusekörper 12 und der Gehäusedeckel 14 sind flüssigkeitsdicht miteinander verbunden, beispielsweise miteinander verschraubt, verklebt oder verriegelt. Die Abwasser-Tauchsonde 10 ist dazu bestimmt, dauerhaft in Abwasser eingetaucht betrieben zu werden.

An der Innenseite des Gehäusedeckels 14 ist in einer Mulde 16 ein Sensor 18 angeordnet, der als Ultraschall-Sensor ausgebildet ist. Der Boden der Mulde 16 bildet ein Sensorfenster 20, das so dünn ist, dass die von dem Ultraschall-Sensor 18 gesendeten und empfangenen Ultraschallwellen in beiden Richtungen nahezu ungehindert hindurchtreten können.

Der Ultraschall-Sensor 18 ist relativ empfindlich gegenüber Feststoffen, die sich an der Außenseite des Sensorfensters 20 anlagern. Aus diesem Grund ist ein drehbarer Wischer 22 vorgesehen, der mit oszillierenden Wischbewegungen das Sensorfenster 20 außenseitig regelmäßig reinigt. Der Wischer 22 ist nicht über eine Welle, sondern über eine magnetische Kopplung mit einem elektrischen Antriebsmotor 24, der innerhalb des Gehäuses 11 angeordnet ist, gekoppelt.

Der Wischer 22 besteht im wesentlichen aus einem Wischerarm 26 mit einem Wischerblatt 28 und einem Wischer-Ringkörper 30, der auf einem Topf 32 sitzt und diesen umgibt. Der Topf 32 ist einstückig mit dem Gehäusedeckel 14 ausgebildet und ragt distal und axial von diesem nach unten hervor. Sowohl der Topf 32 als auch der Ringkörper 30 sind im wesentlichen zylindrisch ausgebildet. Der Gehäusedeckel 14 ist öffnungsfrei ausgebildet.

Die magnetische Kopplung des Wischers 22 mit einem feststehenden Antriebsmotor 24 erfolgt durch dauer-magnetische Elemente 34, 35, 36, 37, die fest verankert in dem Ringkörper 30 sitzen bzw. auf einem drehbar gelagerten Drehkranz 38 in dem Topf 32 angeordnet sind.

Der Drehkranz 38 wird oszillierend bewegt durch den elektrischen Antriebsmotor 24, der ortsfest in dem Gehäuse befestigt ist über seine Antriebsmotorwelle direkt und koaxial mit dem Drehkranz 38 verbunden ist. Der Antriebsmotor 24 führt über die Antriebswelle eine oszillierende Schwenkbewegung aus. Der Drehkranz kann alternativ auch über einen durch einen Antriebsmotor angetriebenen Exzenter oszillierend angetrieben werden.

Die Tauchsonde kann grundsätzlich auch als optische Sonde zur Ermittlung der Schlammspiegelhöhe, der Trübung oder des Nitratgehaltes ausgebildet sein. Grundsätzlich kann die Abwasser-Tauchsonde Parameter des Abwassers bestimmen, die durch ein Sensorfenster hindurch bestimmt werden, das für die genaue Funktion des Sensors freigehalten werden muss von Feststoffen und Verunreinigungen jeder Art.

Grundsätzlich reicht wischerseitig und antriebsmotorseitig ein einziges dauermagnetisches Element aus. Es sollten aber für eine gute Führung jeweils mindestens zwei dauer-magnetische Elemente 34, 35, 36, 37 auf jeder Seite vorgesehen werden, um eine relativ gleichmäßige radiale Kraftverteilung über den gesamten Umfang zu realisieren. Technisch sinnvoll ist ein Vielfaches von 2 als Anzahl an magnetischen Elementen. Die dem Wischer 22 zugeordneten dauer-magnetischen Elemente 34, 35 sind, bezogen auf die Radialen, gegensinnig gepolt zu den jeweils radial gegenüberliegenden dauer-magnetischen Elementen 36, 37, die mit dem Antriebsmotor 24 gekoppelt sind. Ihre Anordnung ist jeweils kranzartig und kann beispielsweise über den Umfang mit alternierender Polarität realisiert sein. Die magnetischen Elemente 36, 37 des Wischers 22 sind außen durch einen ferromagnetischen geschlossenen Rückschluss-Ring 88 miteinander verbunden, der den magnetischen Fluss bündelt und lenkt.

Der Wischer-Ringkörper 30 wird durch eine kreisscheibenförmige Verriegelungsscheibe 50 radial geführt, die mit einem zentralen axialen Gewindebolzen 52 in einer Gewindebohrung 54 des Topfes 32 eingeschraubt ist. Die Verriegelungsscheibe 50 weist proximal Rastnasen 56 auf, die in entsprechenden Rastmulden 58 auf der ringscheibenförmigen Stirnseite 60 des Topfes 32 eingerastet sind. Die Verriegelungsscheibe 50 weist einen Griffsteg 51 auf, so dass die Verriegelungsscheibe 50 unter Überwindung der Rastkräfte manuell entriegelt bzw. verriegelt werden kann.

Radial außen des Ringkörpers 30 ist ein kreisringförmiger Spoiler 64 vorgesehen, der als umlaufende Rippe ausgebildet ist, die axial von dem Gehäusedeckel 14 hervorragt und eine kreisförmige Spaltöffnung zwischen dem Wischer-Ringkörper 30 und dem Gehäusedeckel 14 radial überdeckt. Der Spoiler 64 lenkt insbesondere faserförmige Feststoffe von der Spaltöffnung ab, so dass ihr Eindringen in die Spaltöffnung und ein Verklemmen des Wischer-Ringkörpers 30 vermieden wird.

Die oszillierende Bewegung des Wischers 22 wird durch zwei wischerseitige Anschlagnasen 70, 71 und einen deckelseitigen Anschlagsteg 72 mechanisch begrenzt.

Der Wischer-Ringkörper 30 ist mit einem geringen Radialspiel bzw. Axialspiel beabstandet gegenüber dem Topf 32 bzw. der Verriegelungsscheibe 50. Die radiale Führung des Wischer-Ringkörpers 30 erfolgt ausschließlich durch die Verriegelungsscheibe 50 auf einer linien- und kreisförmigen Berührungslinie, da der Außenrand der Verriegelungsscheibe 50 entsprechend konvex gewölbt ist. Hieraus ergeben sich eine geringe Berührungsfläche und relativ geringe Reibungskräfte und -momente, die durch die magnetische Kopplung überwunden werden müssen.

Die dauer-magnetischen Elemente 34, 35 des Wischer-Ringkörpers 30 einerseits und die dauer-magnetischen Elemente 36, 37 des Drehkranzes 38 andererseits sind axial mindestens um wenige Millimeter versetzt zueinander angeordnet. Die dauer-magnetischen Elemente 34, 35 des Wischer-Ringkörpers 30 sind axial distaler angeordnet als die dauer-magnetischen Elemente 36, 37 des Drehkranzes 38. Hierdurch wird eine axiale Vorspannung des Wischer-Ringkörpers 30 in proximaler Richtung erzeugt, durch die der Wischer-Ringkörper 30 mit seiner proximalen kreisringförmigen Absatzfläche 77 auf eine kreisringförmige Fläche des Gehäusedeckels 14 gedrückt wird. Durch die magnetische Axial-Vorspannung wird bei relativ geringer Reibung eine axiale Lagerung des Wischer-Ringkörpers 30 realisiert, die dennoch kleine Kippbewegungen des Wischerarmes 26 erlaubt, wenn dieser auf ein Hindernis stößt.

Zur Lageerkennung ist an der Antriebsmotorwelle eine Dekoderscheibe 84 befestigt, die von einem optischen Sensor 85 gelesen wird. Durch diese Anordnung lässt sich die rotatorische Lage bzw. Endlage der Antriebsmotorwelle bzw. des Wischers 22 feststellen. Alternativ oder ergänzend können der Wischerarm 26 zur Lageerkennung an seinem distalen Ende einen Permanentmagneten 81 und der Gehäusedeckel 14 an einer Wischer-Endposition oder einer anderen definierten Position des Wischers 22 einen Hall-Geber 80 aufweisen. Hierdurch wird ein Referenzpunkt geschaffen, der bei jedem Wischzyklus durch den Wischer selbst passiert wird, und jederzeit eine genaue Einstellung des durch den Wischer 22 bestrichenen Wischfeldes erlaubt.

## Patentansprüche

1. Abwasser-Tauchsonde (10) mit einem flüssigkeitsdichten Gehäuse (11) mit einem Sensor (18) in dem Gehäuse (11) und einem Sensorfenster (20) an dem Gehäuse (11), wobei
dem Sensorfenster (20) außenseitig ein Wischer (22) zur Reinigung des Sensorfensters (20) zugeordnet ist, und
der Wischer (22) durch einen in dem Gehäuse (11) angeordneten Antriebsmotor (24) angetrieben wird,
**dadurch gekennzeichnet,**
**dass** das Gehäuse (11) öffnungsfrei ist,
**dass** der Antriebsmotor (24) ein magnetisches Element (36, 37) in dem Gehäuse (11) antreibt, und
**dass** dem Wischer (22) ein magnetisches Element (34, 35) außerhalb des Gehäuses (11) zugeordnet ist, derart, dass das magnetische Antriebsmotor-Element (36,37) ein das Gehäuse (11) durchdringendes Magnetfeld erzeugt und wellenlos ein Antriebsmoment auf das magnetische Wischer-Element (34, 35) überträgt.

2. Abwasser-Tauchsonde nach Anspruch 1, **dadurch gekennzeichnet, dass** das Antriebsmotor-Element (36, 37) und das Wischer-Element (34, 35) passivmagnetische Elemente sind.

3. Abwasser-Tauchsonde nach Anspruch 1, **dadurch gekennzeichnet, dass** das Antriebsmotor-Element ein aktives magnetisches Element und das Wischer-Element ein passiv-magnetisches Element ist.

4. Abwasser-Tauchsonde nach einem der Ansprüche 1-3, **dadurch gekennzeichnet, dass** die Wischerbewegung oszillierend ist, und dem Gehäuse (11) und dem Wischer (22) Anschlagelemente (70, 71, 72) zugeordnet sind, die die oszillierende Wischbewegung des Wischers (22) begrenzen.

5. Abwasser-Tauchsonde nach einem der Ansprüche 1-4, **dadurch gekennzeichnet, dass** der Wischer (22) einen die magnetischen Elemente (34, 35) enthaltenden geschlossenen Ringkörper (30) aufweist, der einen die motorseitigen Elemente (36, 37) enthaltenden Topf (32) umgibt.

6. Abwasser-Tauchsonde nach einem der Ansprüche 1-5, **dadurch gekennzeichnet, dass** dem Gehäuse (11) ein Spoiler (64) zugeordnet ist, der eine Spaltöffnung zwischen dem Wischer-Ringkörper (30) und dem Gehäuse (11) radial überdeckt.

7. Abwasser-Tauchsonde nach Anspruch 5 oder 6, **dadurch gekennzeichnet, dass** zwischen dem Wischer-Ringkörper (30) und dem Topf (32) ein Radialspiel vorgesehen ist, wobei die radiale Führung ausschließlich durch eine Verriegelungsscheibe (50) erfolgt, die auf dem Topf (32) axial zentral befestigt ist.

8. Abwasser-Tauchsonde nach einem der Ansprüche 1-7, **dadurch gekennzeichnet, dass** der Wischer-Ringkörper (30) durch die magnetischen Elemente (34, 35, 36, 37) des Wischers (22) und des Motors (24) axial vorgespannt ist.

9. Abwasser-Tauchsonde nach Anspruch 7, **dadurch gekennzeichnet, dass** die Verriegelungsscheibe (50) und der Topf (32) Rastelemente (56, 58) aufweisen, durch die die Verriegelungsscheibe (50) in ihrer Verriegelungsposition an dem Topf (32) verrastet ist.

## Claims

1. A waste water immersion probe (10) comprising a liquid-tight housing (11) with a sensor (18) in the housing (11) and a sensor window (20) at the housing (11), wherein
the sensor window (20) is associated with a wiper (22) on its outer side, for cleaning the sensor window (20), and
the wiper (22) is driven by a drive motor (24) arranged in the housing (11),
**characterized in that**
the housing (11) is devoid of openings,
the drive motor (24) drives a magnetic element (36, 37) in the housing (11), and
the wiper (22) is associated with a magnetic element (34, 35) outside the housing (11) such that the magnetic drive motor element (36, 37) generates a magnetic field penetrating the housing (11) and transmits a drive torque to the magnetic wiper element (34, 35) without a shaft.

2. The waste water immersion probe of claim 1, **characterized in that** the drive motor element (36, 37) and the wiper element (34, 35) are passive magnetic elements.

3. The waste water immersion probe of claim 1, **characterized in that** the drive motor element is an active magnetic element and the wiper element is a passive magnetic element.

4. The waste water immersion probe of one of claims 1-3, **characterized in that** the wiper movement is oscillating, and that the housing (11) and the wiper (22) are associated with stop elements (70, 71, 72) that limit the oscillating wiping movement of the wiper (22).

5. The waste water immersion probe of one of claims 1-4, **characterized in that** the wiper (22) comprises a closed annular body (30) containing the magnetic elements (34, 35), the annular body surrounding a can (32) containing the motor-side elements (36, 37).

6. The waste water immersion probe of one of claims 1-5, **characterized in that** the housing (11) is associated with a spoiler (64) radially covering a gap opening between the annular wiper body (30) and the housing (11).

7. The waste water immersion probe of claim 5 or 6, **characterized in that** a radial play is provided between the annular wiper body (30) and the pot (32), the radial guiding being effected exclusively by a locking disc (50) mounted centrally in the axial direction on the can (32).

8. The waste water immersion probe of one of claims 1-7, **characterized in that** the annular wiper body (30) is biased axially by the magnetic elements (34, 35, 36, 37) of the wiper (22) and the motor (24).

9. The waste water immersion probe of claim 7, **characterized in that** the locking disc (50) and the can (32) have locking elements (56, 58) by which the locking disc (50) is locked to the pot (32) in its locked position.

## Revendications

1. Sonde immergée pour eaux usées (10) avec un boitier (11) étanche à fluide comprenant un capteur (18) dans ledit boitier (11) et une fenêtre de capteur (20) dans ledit boiter (11), dans laquelle
ladite fenêtre de capteur (20) est associée à un essuyeur (22), prévu sur l'extérieur, pour nettoyer ladite fenêtre de capteur (20), et
ledit essuyeur (22) est entrainé par un moteur d'entrainement (24) disposé dans ledit boitier (11),
**caractérisée en ce que**
ledit boitier (11) est dépourvu d'ouvertures,
ledit moteur d'entrainement (24) entraine un élément magnétique (36, 37) dans ledit boitier (11), et
l'essuyeur (22) est associé à un élément magnétique (34, 35) hors dudit boitier (11), de sorte que ledit élément magnétique (36, 37) dudit moteur d'entrainement génère un champ magnétique pénétrant ledit boitier (11) et transmet, sans arbre, un moment d'entrainement audit élément magnétique (34, 35) dudit essuyeur.

2. Sonde immergée pour eaux usées selon la revendication 1, **caractérisée en ce que** ledit élément magnétique (36, 37) dudit moteur d'entrainement et ledit élément magnétique (34, 35) dudit essuyeur sont des éléments magnétiques passifs.

3. Sonde immergée pour eaux usées selon la revendication 1, **caractérisée en ce que** ledit élément dudit moteur d'entrainement est un élément magnétique actif et ledit élément dudit essuyeur est un élément magnétique passif.

4. Sonde immergée pour eaux usées selon l'une quelconque des revendications 1-3, **caractérisée en ce que** le mouvement dudit essuyeur est oscillant, et que le boitier (11) et l'essuyeur (22) sont associés à des éléments de butée (70, 71, 72) limitant ledit mouvement d'essuyage oscillant dudit essuyeur (22).

5. Sonde immergée pour eaux usées selon l'une quelconque des revendications 1-4, **caractérisée en ce que** ledit essuyeur (22) comprend un corps annulaire fermé (30) contenant lesdits éléments magnétiques (34, 35) et entourant un pot (32) contenant les éléments côté moteur (36, 37).

6. Sonde immergée pour eaux usées selon l'une quelconque des revendications 1-5, **caractérisée en ce que** ledit boitier (11) est associé à un becquet (64), qui couvre radialement une fente entre ledit corps annulaire d'essuyeur (30) et ledit boitier (11).

7. Sonde immergée pour eaux usées selon la revendication 5 ou 6, **caractérisée en ce qu'**un jeu radial est prévu entre ledit corps annulaire d'essuyeur (30) et ledit pot (32), le guidage radial étant effectué exclusivement par un disque de verrouillage (50) fixé centralement dans la direction axiale sur le pot (32).

8. Sonde immergée pour eaux usées selon l'une quelconque des revendications 1-7, **caractérisée en ce que** ledit corps annulaire d'essuyeur (30) est précontraint axialement par lesdits éléments magnétiques (34. 35, 36, 37) dudit essuyeur (22) et dudit moteur (24).

9. Sonde immergée pour eaux usées selon la revendication 7, **caractérisée en ce que** le disque de verrouillage (50) et le pot (32) comprennent des éléments d'encoche (56, 58) par lesquels le disque de verrouillage (50) est enclenché avec le pot (32) dans la position de verrouillage du disque.
